# EUROPEAN PATENT SPECIFICATION

(11) **EP 1 457 781 B1**
(45) Date of publication and mention of the grant of the patent: **09.06.2010**
(21) Application number: 04005789.5
(22) Date of filing: 11.03.2004
(51) Int. Cl.: G01N 33/50, G01N 33/569, G01N 33/14

(54) **Simultaneous determination of cell proliferation inhibition activity and toxicity using flow cytometry**
Gleichzeitigen flusszytometrischer Messungen von die Inhibition der Zellproliferation und der Toxizität
Détection simultanée par cytométrie de flux de l' inhibition de la prolifération cellulaire ainsi que la toxicité

(30) Priority: 12.03.2003 EP 03005335
(43) Date of publication of application: 15.09.2004
(73) Proprietor: F. Hoffmann-La Roche AG, 4070 Basel (CH)
(72) Inventor: Goller, Bernhard, 82377 Penzberg (DE); Kubbies, Manfred, 82377 Penzberg (DE)
(74) Representative: Schreiner, Siegfried

(56) References cited:
- US-B1- 6 403 378
- STEWART C C ET AL: "QUANTITATION OF CELL CONCENTRATION USING THE FLOW CYTOMETER" CYTOMETRY, vol. 2, no. 4, 1982, pages 238-243, XP008021119 ISSN: 0196-4763
- STEVENSON A P ET AL: "SIMULTANEOUS MEASUREMENTS OF MACROPHAGE-INDUCED CYTOSTASIS AND CYTOTOXICITY OF EMT-6 CELLS BY FLOW CYTOMETRY" CANCER RESEARCH, vol. 46, no. 1, 1986, pages 99-105, XP008021077 ISSN: 0008-5472
- CAMPBELL D L ET AL: "Flow cytometric technique for quantitating cytotoxic response to photodynamic therapy." PHOTOCHEMISTRY AND PHOTOBIOLOGY, vol. 63, no. 1, 1996, pages 111-116, XP008021003 ISSN: 0031-8655
- VERHOEF V ET AL: "RHODAMINE 123 AND FLOW CYTOMETRY TO MONITOR THE CYTOTOXIC ACTIONS OF NUCLEOSIDE ANALOGUES IN NONDIVIDING HUMAN LYMPHOCYTES" ANTICANCER RESEARCH, vol. 6, no. 5, 1986, pages 1117-1122, XP008021053 ISSN: 0250-7005
- DATABASE BIOSIS [Online] BIOSCIENCES INFORMATION SERVICE, PHILADELPHIA, PA, US; July 2001 (2001-07), SNYDER EDWARD L ET AL: "Ex vivo evaluation of PBMNCs collected with a new cell separator." XP002252252 Database accession no. PREV200100412978 & TRANSFUSION (BETHESDA), vol. 41, no. 7, July 2001 (2001-07), pages 940-949, ISSN: 0041-1132

## Description

The invention provides a method for the simultaneous determination of cell proliferation inhibition activity and toxicity of a substance which can be performed within the screening of such a substance for its activity in proliferation inhibition.

### Background of the Invention

The identification of substances which are potent proliferation inhibitors is of great importance during drug discovery, preferably in the field of oncology. There exists a variety of in vitro cell-based assays for the investigation of the antiproliferative effect of such candidate substances. Examples of such assays are, for instance, the MTT colorimetric assay, the [3H] thymidine uptake assay, and the WST assay (Johnston, P., in: Cellular Assays in HTS, Methods in Molecular Biology, Vol. 110, Janzen W.P. (ed.), Humana Press, NJ, pp.107-116; Bellamy, W.T., Drugs 44 (1992) 690-708). In regard to drug development, it is necessary that such assays can be used in high throughput screening of large numbers of potential drug candidates.

For the determination of the proliferation status of hematopoietic cells, it is known to contact the cell population with the substance to be investigated and a reagent capable of generating luminescence in the presence of ATP and detecting the luminescence as a measure of the proliferation status (US 2002/0146680). An assay for determining the number of cells in cell culture by fluorescence measurement is described in US Patent No. 5,972,639.

However, such assays are time-consuming, provide only limited results, and do not discriminate between inhibition of proliferation and induction of cell death.

Therefore, there exists a need for an assay which allows the determination of the influence of a substance on the proliferation and on the induction of cell death in a simple way and simultaneously. Moreover, there is a need that such assays can be performed automatically.

Chandrasekaran et al (1995. Canc Chemother Pharmacol, 35, 489-495) report the flow cytometric determination of the cytostatic and cytotoxic effect of 3'-azido-3'-deoxythymidine. Distinction between cytostasis and cytotoxicity is made with further experiments involving a. o. BrdRD incorporation. Two discrete samples are processed separately.

### Summary of the Invention

The invention provides a method for the simultaneous determination of cell proliferation inhibition activity and cell toxicity (induction of cell death) of a low molecular weight chemical compound using a proliferating mammalian cell sample as a test system, characterized by
a) treating said cell sample as a cell suspension or as adherent cells in a predetermined volume with said compound in at least two different predetermined concentrations;
b) treating said cell sample with a first fluorescence dye staining specifically either dead cells or viable cells and optionally with a second fluorescence dye staining all cells;
c) adding to said cell sample a predetermined amount of latex particles with a size ranging from about 1 to about 20 µm to said volume and optionally staining said latex particles with a third fluorescence dye;
d) determining the ratio of the amount of total cells to the amount of latex particles in said sample;
e) determining with the results of step d) and by flow cytometric analysis in said sample
   - the number of dead cells or viable cells by fluorescence light emitted by said first fluorescence dye at a first wavelength;
   - the number of total cells per volume by scatter light at a first angle or alternatively by fluorescence light emitted by said second fluorescence dye at a second wavelength;
   - the number of latex particles per volume by scatter light at a second angle or alternatively by fluorescence light emitted by said third fluorescence dye at a third wavelength;
f) and determine with the results of step d) and e) cell proliferation activity and toxicity of said compound.

In a preferred embodiment of the invention, the dead cells are specifically stained by a fluorescence dye and the number of latex particles and cells is measured by differential side scatter light and forward scatter light.

In a further preferred embodiment of the invention, the cells are human CD34⁺ progenitor cells.

In a preferred embodiment of the invention, the method is performed in at least five different concentrations of said substance to be investigated, which preferably enables the calculation of IC₅₀ values for proliferation and induction of cell death. Preferably, the concentration range is of a factor of about 1 to 10,000.

After treating the cells with the substance to be investigated, the cells are cultivated under standard conditions that would allow cell proliferation. In a preferred embodiment of the invention, the cells are cultivated in parallel in multiple devices, preferably in multi-well microtiter plates. In addition, also the substances to be investigated are added to these devices in the different concentrations, preferably by an automatic pipetting means.

### Detailed Description of the Invention

A delayed cell growth during cultivation of cells in the presence of a substance to be suspected as a cell inhibitor can be caused by cell death and/or cell proliferation inhibition. Therefore, from a mere determination of the amount of cells after cultivation it is not possible to conclude directly toxicity and/or inhibition activity of the substance. It is necessary to know the amounts of viable and dead cells and their ratio in regard to the total cell amount for such a conclusion. The invention provides a method for simultaneous determination of these parameters in a rapid manner. In addition, the method according to the invention can be performed automatically and therefore allows a high throughput investigation of toxicity and proliferation inhibition activity of substances.

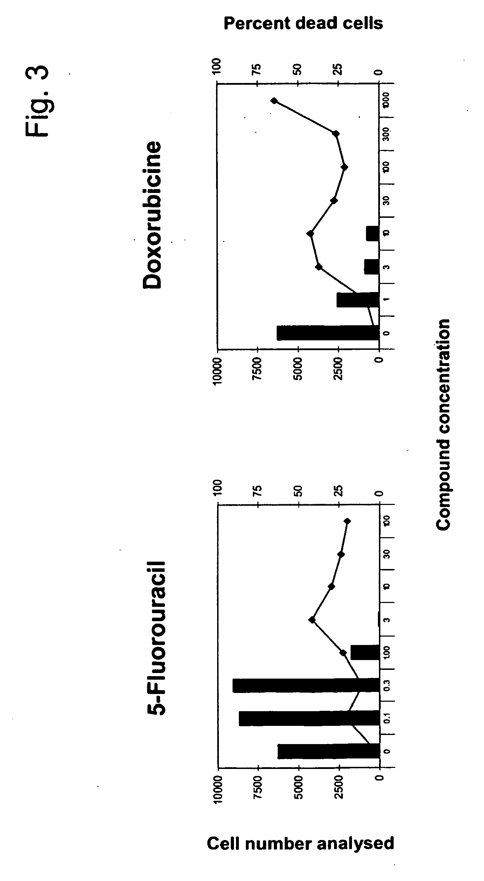

According to the invention, a defined (predetermined) aliquot of latex particles is added to each cell cultivation device. Based on the counting of the latex particles during flow cytometric analysis, the volume of the liquid in which all the counting were performed can be estimated. Therefore, the invention provides a method for simultaneous determination of the amount of total cells per volume (which is a measure of cell proliferation) and the amount of viable cells per volume after cultivation in the presence of a substance suspected to be a proliferation inhibitor and/or to be toxic to the cells of the test system. The comparison of the amount of total cells with the amount of dead cells or viable cells for different concentrations of the substance provides the information on the relation between inhibition of cell proliferation and toxicity of the substance. This can be deduced immediately from Fig. 3. If the percentage of dead cells to total cells increases significantly whereas the number of total cells decreases, this indicates high toxicity of the substance. If, however, the percentage of dead cells to total cells does not increase significantly with increasing substance concentration whereas the number of total cells decreases, then the substance shows a low toxicity. Examples of substances with high toxicities are shown in Fig. 3. 5-fluorouracil and doxorubicine are substances which display toxicity in parallel to significant proliferation inhibition. For 5-fluorouracil the percentage of dead cells to total cells is in the range between 0 and 25% for the concentration range of the substance in which proliferation inhibition is found as more than 50%. For doxorubicine the percentage of dead cells increases with substance concentration up to 40% and even higher whereas proliferation inhibition is about 80%.

The cultivation process of the proliferating cells, e.g. CD34 cells, is performed as described below. Prior to FACS analysis the fluorescence dye for staining the viable or dead cells and a distinct number of latex beads are added to each well device (preferably microtiter plates, or the like) for staining of viable or dead cells and counting of latex particles as a measure of cell culture medium volume analyzed, respectively. After adding the substances to be investigated and after performing the cell cultivation in the different devices, the microtiter plate is placed onto an automated pipetting instrument which routes aliquots of the cell sample from each well through the FACS flow chamber. A distinct number of latex beads is being analyzed (thereby giving information about the medium volume measured), measurement is stopped and the pipetting robot takes aliquots from the next well, and analysis starts again as before.

The term "substances to be investigated" are substances, which is suspected of having proliferation inhibition or cytotoxic activity. These substances are low molecular weight chemical compounds. The compounds are used in at least two different concentrations, preferably, however, in more concentrations, for example five different concentrations or more. The reasons for this is that based on the results for such different concentrations, it is possible to investigate the concentration dependent proliferation inhibition and cell death induction very easily and decide whether the substance inducts cell death considerably and/or inhibits proliferation. For the comparison of these values, especially for the comparison of the concentration dependence of proliferation and induction of cell death, it is possible to identify potential candidate substances which show for example strong proliferation inhibition without considerable induction of cell death. In addition, performing the method of the invention at different concentrations allows to calculate IC₅₀ values both for proliferation ("proliferation activity") and induction of cell death ("toxicity"). The concentrations and the range of concentrations depend on the desired potency of the substance to be investigated and also on the type of cell used for the investigation. Usually, the concentration range is in the micromolar range and between about 0.01 µM/ml and 100 µM/ml.

The term "cell" as used herein describes such proliferating mammalian cells which are useful as a test system for proliferation assays. Such cells are well-known in the state of the art and are, for example, human CD34⁺ progenitor and stem cells, lymphocytes, normal fibroblasts/keratinocytes, normal endothelial- and epithelial cells, artifical transformed mammalian cells, leukemic cells and solid tumor cells. The proliferation of such cells can be inhibited by known inhibitors such as paclitaxel, doxorubicine or 5-fluorouracil.

Usually, the cells are seeded in parallel devices and propagated in suspension. Usually, and, for example, if 96 well microtiter plates are used for the cultivation of CD34 cells, 100 to 400 cells/200 µl are seeded per well and propagated preferably until they have reached about 10,000 cells/200 µl per well under standard conditions (without substance to be investigated). For cultivation of the cells, conventional cultivation media, preferably with added growth factors, are used.

The term "latex particles" as used herein describes such latex particles as are widely used in calibrating fluids for automatic instruments for cell counting. Such latex particles are described, for example, in US Patent No. 3,977,995. Such latex particles usually consist of a synthetic latex made from polystyrene polyvinyl toluene or styrene divinylbenzene copolymer. The particle size usually ranges from 1 to about 20 µm and the particles are added to the cell suspension in an amount of 10,000 particles/200 µl.

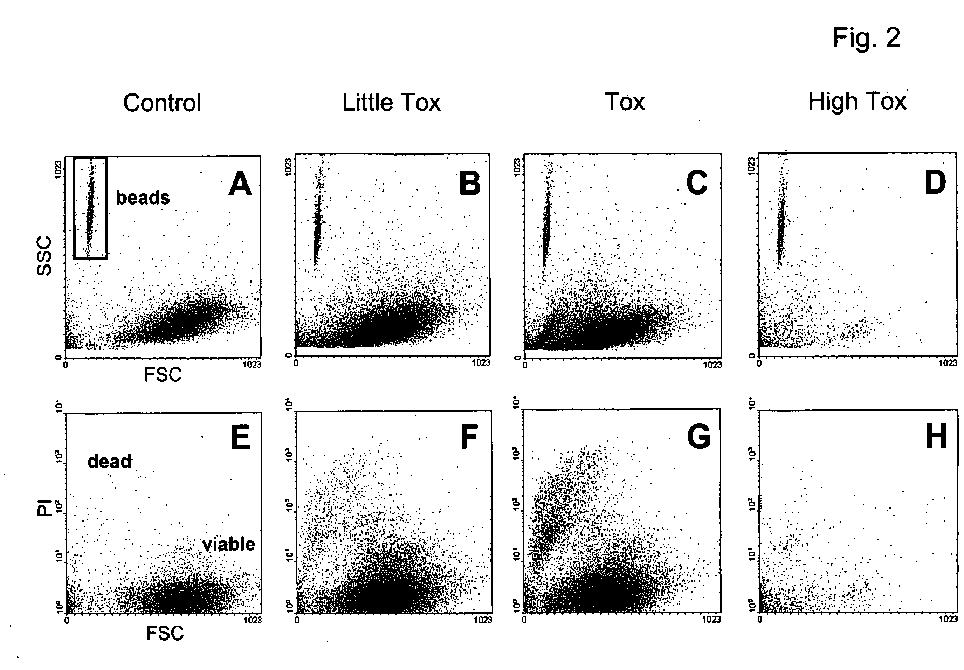

The measurement of the parameters for the determination according to the invention is performed by flow cytometric analysis in an apparatus wherein fluorescence at different wavelengths and scatter light at different angles can be measured and correlated with the amount of cells providing such signals. The parameters are determined while the cells from one sample are flowing through the analytical means of the apparatus either in parallel or immediately one parameter after another (simultaneous determination). Preferably, such a determination is performed by the use of an analytical FACS instrument. Scatter light is measured at different angles, preferably as forward scatter light (FSC) (maximum scatter light angle up to 20°, preferably lower) and side scatter light (SSC) (angle about 90°). Though cells and latex particles might not differ considerably in their size, their behavior in relation to light scatter is considerably different, presumably based on the internal structure and shape of the cells as well as on their different refractory index. Based on this difference, the cells (viable/proliferating, viable arrested cells and dead cells) can be easily discriminated from the latex particles by a considerably different scatter light angle. SSC refers to the latex beads and FSC refers to cells (cf. Fig. 2a). However, it is also possible to stain all cells or all latex particles with fluorescence dyes and discriminate latex particles and cells by the investigation of fluorescence light at different wavelengths. The use of the parameters measured depends simply on the possibilities and convenience provided by the apparatus used. If an apparatus is able to measure fluorescence light simultaneously at three or four different fluorescence wavelengths, latex particles, all cells and either the dividing or dead cells can be labeled specifically with fluorescence dyes. Therefore, all combinations of scatter light and fluorescence light can be used for the measurement of the parameters with the provision that the scatter light parameter is not useful for a direct discrimination of latex particles and dead cells of viable and proliferating and of viable and arrested cells.

The term "fluorescence dye staining specifically dead cells or viable cells" as used herein describes dyes which enter only dead cells (e.g. propidium iodide), or which enrich only in viable cells (e. g. fluoresceindiacetate). If cell counting of all cells is required, fluorescent dyes are useful for staining dead cells as well as for staining viable cells (e.g. Hoechst 33342). Numerous fluorescence dyes useful for selective labeling of viable or dead cells are described (e.g. catalog from Molecular Probes; http://www.molecularprobes.com/). The fluorescence dyes are added in an appropriate amount, which is, for example, 1 µg/ml final concentration.

The following examples, reference listing and figures are provided to aid the understanding of the present invention, the true scope of which is set forth in the appended claims. It is understood that modifications can be made in the procedures set forth without departing from the spirit of the invention.

### Description of the Figures

- **Figure 1**: Microscopic analysis of cell growth and cell death in CD34 cell populations. Cytokine stimulated CD34 cell were cultivated for 4 and 7 days untreated or treated with a cytotoxic compound.
- **Figure 2**: FACS analysis of standard beads, and viable and dead cytokine stimulated CD34 cells cultivated for 10 days. Cell were either untreated (left row) or treated with cytotoxic drug inducing different levels of cytotoxicity.
- **Figure 3**: Cell number and percentage of dead cells quantitated from a FACS analysis as shown in Figure 2. Two typical cytotoxic compounds, 5-fluorouracil (0.1 - 100 µmol/l) and doxorubicine (1 - 1000 ng/ml), are displayed. The IC₅₀ value for the cell number analysis corresponds to 0.34 µmol/l and 0.67 ng/ml for 5-fluorouracil and doxorubicine, respectively.

### Example

In a typical experimental setting, human cord blood CD34+ cells are isolated to high purity (>90 %) by immunomagnetic cell sorting, aliquoted and frozen in DMSO containing medium. For cell culture setup, frozen CD34+ cells are thawed and 200 cells are seeded in 200 µl complete medium of a well of a 96 well plate. The basal cell culture medium consists of IMDM-Medium supplemented with 20 % human AB serum and antibiotics (pen/strep). The CD34+ cells are activated by addition of following cytokine cocktail: hu-SCF (100 ng/ml), hu-IL6 (10 ng/ml), hu-GM-CSF (100 U/ml), hu-TPO (25 ng/ml) and hu-EPO (5 U/ml). Cultivation is performed in incubators at 37°C, high humidity and 7 % CO₂.

Two different experimental setup can be used: 1) addition of the inhibitor compound prior supplementation of the medium with cytokines to hit preferentially resting cells, and 2) addition of inhibitor compound 4 days after CD34 activation with cytokines to hit preferentially activated cells.

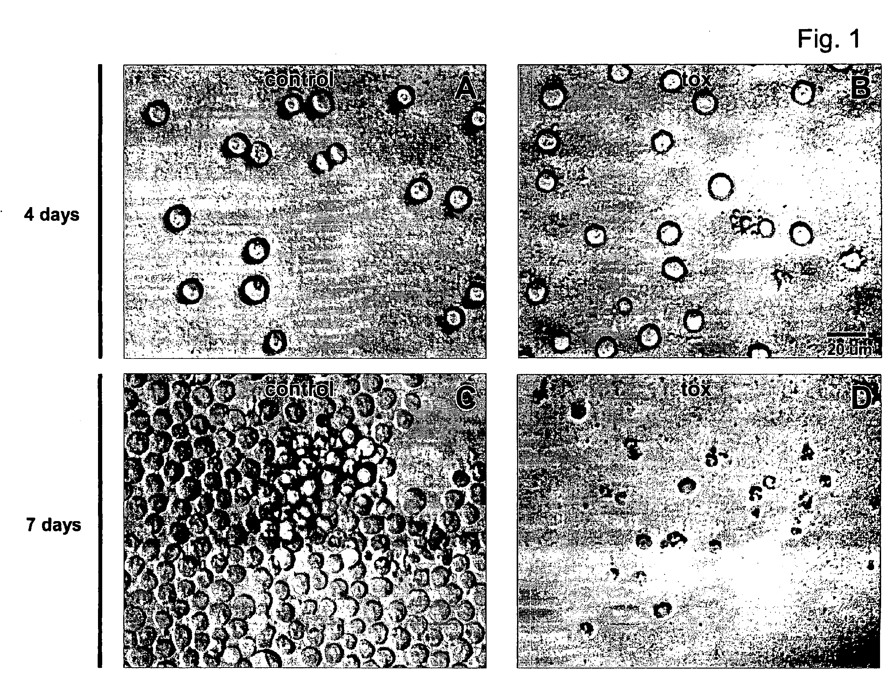

As shown in Figure 1, activated and viable CD34 cells appear as large, spheroid cells after 4 day cultivation period (panelA). The cell number increased significantly when the cells were grown for additional 3 days (panel C). A cytotoxic drug induces cell death in CD34 cells which appear fragmented in microscopic images after a 4 day culture period (panel B), and the number of fragmented cells increases without any significant increase of the cell number (panel D).

Dependent on the proliferation rate of the CD34+ cells, harvest and analysis of cell number and cytotoxicity is performed after 9-10 d cell culture. The cell number and cytotoxicity is analysed by FACS technique. The number of cell present in the well is counted by comparision with an internal bead standard. A aliquot of 10000 beads is added to each well harbouring 200 µl medium volume (FluoSpheres® polystyrene, 15 µm; Molecular Probes). As displayed in Fig. 2, in a typical FACS instrument setting, the viable cells appear in the middle/right position of the forward scatter (FSC) and low side scatter (SSC) but the standard beads added are present in a region with lower FSC and high SSC (panel A). A gate, shown as rectangular region, is set onto the standard beads, a distinct number is being counted, analysis is being stopped and the corresponding volume of medium containg cells is calculated. Panel E displays the viability assay of analyzed CD34 cells of this measurement. The viable cells appear again in the middle/right position of the forward scatter (FSC) displaying low/no propidium iodide labeling. The few dead cells appear left or in the upper/left position relative to to viable cell population. The FACS measurement now takes an aliqout count of the beads and calculates the number of cells being present within the well, whereas the cytotoxicity, the number of dead cells, is recorded in parallel during this measurement (addition of propidium iodide; final concentration 1 µg/ml). With increasing concentrations of a cytotoxic compound the number of cell in the dead cell cluster increases significantly (Fig. 2 panel F and G). At the highest inhibitory and cytotoxic concentration very few cells are present anymore in the viable cell cluster and the majority of cells has been killed as evident by increased PI staining or fragmentation (panel H).

A typical example of the calculation of the number of CD34 cells and percentage of dead cells in a well is shown in Fig. 3 for two cytotoxic compounds, namely 5-fluorouracil and doxorubicine. The cell number per well is displayed as bar for each concentration tested. For 5-fluorouracil the cell number decreases significantly between 0.3 and1.0 µmol/l and at the lowest concentration of 1 ng/ml doxorubicine the cell number is lower already. Applying a IC₅₀ calculation program, the IC₅₀ values for the inhibitor compounds were calculated to be 0.34 µmol/l and 0.67 ng/ml for 5-fluorouracil and doxorubicine, respectively. The increase of the percentage of dead cells obtained from the analysis of the PI positive cell fraction (Fig.2 panels E to H) is shown as solid line.

### List of References

Bellamy, W.T., Drugs 44 (1992) 690-708
Johnston, P., in: Cellular Assays in HTS, Methods in Molecular Biology, Vol. 110, Janzen W.P. (ed.), Humana Press, NJ, pp.107-116
US Patent No. 3,977,995
US Patent No. 5,972,639
US 2002/0146680

## Claims

1. Method for the simultaneous determination of cell proliferation inhibition activity and cell toxicity of a low molecular weight chemical compound using a proliferating mammalian cell sample as a test system, **characterized by**
a) treating said cell sample as a cell suspension or as adherent cells in a predetermined volume with said compound in at least two different predetermined concentrations;
b) treating said cell sample with a first fluorescence dye staining specifically either dead cells or viable cells and optionally with a second fluorescence dye staining all cells;
c) adding to said cell sample a predetermined amount of latex particles with a size ranging from about 1 to about 20 µm to said volume and optionally staining said latex particles with a third fluorescence dye;
d) determining the ratio of the amount of total cells to the amount of latex particles in said sample;
e) determining with the results of step d) and by flow cytometric analysis in said sample
- the number of dead cells or viable cells by fluorescence light emitted by said first fluorescence dye at a first wavelength;
- the number of total cells per volume by scatter light at a first angle or alternatively by fluorescence light emitted by said second fluorescence dye at a second wavelength;
- the number of latex particles per volume by scatter light at a second angle or alternatively by fluorescence light emitted by said third fluorescence dye at a third wavelength;
f) and determine with the results of step d) and e) cell proliferation activity and toxicity of said compound.

2. Method according to claim 1, **characterized in that** the dead cells are specifically stained by a fluorescence dye and the number of latex particles and cells is measured by differential side scatter light and forward scatter light.

3. Method according to claim 1 or 2, **characterized in that** the cells are human CD34⁺ progenitor cells.

4. Method according to claims 1 to 3, **characterized in that** the cells are cultivated in parallel in multiple devices and the samples are transferred to the flow cytometric analysis apparatus by automated pipetting.

## Patentansprüche

1. Verfahren zur gleichzeitigen Bestimmung der inhibitorischen Aktivität auf die Zellproliferation und der Zelltoxizität einer chemischen Verbindung mit geringem Molekulargewicht, unter Verwendung einer proliferierenden Säugetier-Zellprobe als Testsystem, **gekennzeichnet durch**
a) Behandeln der Zellprobe als Zellsuspension oder als adhärente Zellen in einem vorbestimmten Volumen mit der Verbindung in mindestens zwei unterschiedlichen vorbestimmten Konzentrationen;
b) Behandeln der Zellprobe mit einem ersten Fluoreszenzfarbstoff, der entweder tote Zellen oder lebensfähige Zellen spezifisch färbt, und gegebenenfalls mit einem zweiten Fluoreszenzfarbstoff, der alle Zellen färbt;
c) Zufügen zu der Zellprobe einer vorbestimmten Menge an Latexpartikeln mit einer Größe im Bereich von etwa 1 bis etwa 20 µm zu dem Volumen und gegebenenfalls Färben der Latexpartikel mit einem dritten Fluoreszenzfarbstoff;
d) Bestimmen des Verhältnisses der Gesamtmenge an Zellen zu der Menge an Latexpartikeln in der Probe;
e) Bestimmen
- der Anzahl an toten Zellen oder lebensfähigen Zellen mittels Fluoreszenzlicht, das von dem ersten Fluoreszenzfarbstoff bei einer ersten Wellenlänge emittiert wird;
- der Gesamtanzahl an Zellen pro Volumen mittels Streulicht unter einem ersten Winkel oder alternativ mittels Fluoreszenzlicht, das von dem zweiten Fluoreszenzfarbstoff bei einer zweiten Wellenlänge emittiert wird;
- der Anzahl an Latexpartikeln pro Volumen mittels Streulicht unter einem zweiten Winkel oder alternativ mittels Fluoreszenzlicht, das von dem dritten Fluoreszenzfarbstoff bei einer dritten Wellenlänge emittiert wird;
mit den Ergebnissen aus Schritt d) und mittels durchflusszytometrischer Analyse in der Probe;
f) und Bestimmen der Aktivität der Zellproliferation und der Toxizität der Verbindung mit den Ergebnissen aus Schritt d) und e).

2. Verfahren nach Anspruch 1, **dadurch gekennzeichnet, dass** die toten Zellen durch einen Fluoreszenzfarbstoff spezifisch gefärbt werden, und dass die Anzahl an Latexpartikeln und Zellen mittels unterschiedlichem Seitwärtsstreulicht und Vorwärtsstreulicht gemessen wird.

3. Verfahren nach Anspruch 1 oder 2, **dadurch gekennzeichnet, dass** die Zellen humane CD34⁺-Vorläuferzellen sind.

4. Verfahren nach den Ansprüchen 1 bis 3, **dadurch gekennzeichnet, dass** die Zellen parallel in mehreren Vorrichtungen kultiviert werden, und dass die Proben durch automatische Pipettierung an das Gerät für die durchflusszytometrische Analyse überführt werden.

## Revendications

1. Procédé pour déterminer simultanément l'activité inhibitrice sur la prolifération cellulaire et la toxicité sur les cellules d'un composé chimique de poids moléculaire bas en utilisant un échantillon de cellules de mammifère en prolifération comme système d'essai, **caractérisé par**
a) le traitement dudit échantillon de cellules sous la forme d'une suspension de cellules ou sous la forme de cellules adhérentes dans un volume prédéterminé avec ledit composé à au moins deux concentrations prédéterminées différentes ;
b) le traitement dudit échantillon de cellules avec un premier colorant fluorescent colorant spécifiquement soit les cellules mortes, soit les cellules viables et facultativement avec un deuxième colorant fluorescent colorant toutes les cellules ;
c) l'ajout audit échantillon de cellules d'une quantité prédéterminée de particules de latex ayant une taille située dans la plage allant d'environ 1 µm à environ 20 µm audit volume et facultativement la coloration desdites particules de latex avec un troisième colorant fluorescent ;
d) la détermination du rapport entre la quantité de cellules totales et la quantité de particules de latex dans ledit échantillon ;
e) la détermination avec les résultats de l'étape d) et par une analyse par cytométrie en flux dans ledit échantillon
- du nombre de cellules mortes ou de cellules viables par la lumière de fluorescence émise par ledit premier colorant fluorescent à une première longueur d'onde ;
- du nombre de cellules totales par volume par la lumière diffusée à un premier angle ou en variante par la lumière de fluorescence émise par ledit deuxième colorant fluorescent à une deuxième longueur d'onde ;
- du nombre de particules de latex par volume par la lumière diffusée à un second angle ou en variante par la lumière de fluorescence émise par ledit troisième colorant fluorescent à une troisième longueur d'onde ;
f) et la détermination avec les résultats de l'étape d) et de l'étape e) de l'activité sur la prolifération cellulaire et de la toxicité dudit composé.

2. Procédé selon la revendication 1, **caractérisé en ce que** les cellules mortes sont spécifiquement colorées par un colorant fluorescent et le nombre de particules de latex et de cellules est mesuré par différentiel de la lumière diffuse latérale et la lumière diffuse avant.

3. Procédé selon la revendication 1 ou 2, **caractérisé en ce que** les cellules sont des cellules progénitrices CD34⁺ humaines.

4. Procédé selon les revendications 1 à 3, **caractérisé en ce que** les cellules sont cultivées en parallèle dans plusieurs dispositifs et les échantillons sont transférés vers l'appareil d'analyse par cytométrie en flux par pipetage automatisé.
